# EUROPEAN PATENT SPECIFICATION

(11) **EP 3 256 095 B1**
(45) Date of publication and mention of the grant of the patent: **28.06.2023**
(21) Application number: 16703994.0
(22) Date of filing: 12.02.2016
(51) Int. Cl.: A61K 8/27, A61K 8/44, A61Q 5/00, A61K 8/67, A61K 8/97

(54) **USE OF A COMBINATION OF TAURINE OR A DERIVATIVE THEREOF, BIOTIN, VITAMIN C, ZINC OR A SALT THEREOF AND GRAPE EXTRACT FOR COMBATING FINE HAIR**
VERWENDUNG EINER KOMBINATION VON TAURIN ODER EINES DERIVATS DAVON, BIOTIN, VITAMIN C, ZINK ODER EINES SALZES DAVON UND TRAUBENEXTRAKT ZUR BEKÄMPFUNG VON FEINEM HAAR
UTILISATION D'UNE ASSOCIATION DE TAURINE OU D'UN DÉRIVÉ DE CELLE-CI, DE BIOTINE, DE VITAMIN C, DE ZINC OU D'UNE DE SES SELS ET D'EXTRAIT DE RAISIN POUR LUTTER CONTRE LES CHEVEUX FINS

(30) Priority: 13.02.2015 FR 1551201
(43) Date of publication of application: 20.12.2017
(73) Proprietor: NUTRICOS Technologies, 92117 Clichy Cedex (FR)
(72) Inventor: PICCARDI, Nathalie, 21310 Arceau (FR)
(74) Representative: Cabinet Nony
(86) International application number: PCT/EP2016/052960
(87) International publication number: WO 2016/128532

(56) References cited:
- FR-A1- 2 861 594
- FR-A1- 2 996 128
- US-A1- 2005 175 565
- DATABASE GNPD [Online] MINTEL; November 2012 (2012-11), Aflofarm Fabryka Leków: "Two Stage Hair Strengthener", XP002751621, Database accession no. 1920318
- DATABASE GNPD [Online] MINTEL; February 2013 (2013-02), Acofarma: "Hair Loss Food Supplement Tablets", XP002751622, Database accession no. 1995655

## Description

The present invention relates to the field of caring for keratin fibres, more particularly the hair. In particular, the invention is directed towards proposing a combination that is useful for improving the quality of the head hair and in particular for the purpose of increasing the thickness of keratin fibres, and more particularly hair fibres.

Hair constitutes a major distinctive element of femininity. This is why the majority of women wish to have healthy, vigorous hair throughout their life.

Thus, women with fine hair may suffer from this situation, insofar as the consequence of this is a head of hair that lacks volume and tonicity, with hair that seems to have a tapered feel. Fine hair is difficult to style and makes hairstyle hold difficult over time.

Furthermore, due to their fineness, fine hairs are more fragile and consequently prove to be more brittle and to have less tensile strength, especially when they undergo a simple mechanical treatment such as brushing, combing or straightening, all the more so since they have a greater tendency to become entangled.

In addition, independently of any hair loss, fine hair results in greater visibility of the scalp and gives an illusion of thinned hair, which is not considered as being aesthetically pleasing by the majority of women.

Women may naturally have fine hair, but this feature may also appear, or become accentuated, with age.

Thus, the object of the invention is to propose a solution to women of all ages for improving the quality of their head of hair and more particularly for combating fine hair, and in particular for increasing the thickness of the hairs.

Many cosmetic compositions containing amino acids, in particular glutamine, arginine and cysteine, or peptides and/or taurine, exist for treating the scalp.

By way of example, WO 2004/000293 describes the use of taurine and/or hypotaurine and/or acceptable salts thereof for the preparation of an oral composition that is useful in the treatment and prevention of aging of the pilosebaceous unit and/or of alopecia.

The product sold under the name Asystor Hair by the company Aflofarm Fabryka Leków (described in document XP002751621) consists of two tablets wherein one of the tablets contains taurine and is used for improving microcirculation of the scalp and maintain hair density.

However, consumers are still in search of active agents or combinations of active agents that are more effective for increasing and/or maintaining the thickness of hair fibres and/or for limiting the thinning of hair fibres and thus combating the problems mentioned previously.

The object of the present invention is to meet these needs.

The present text describes the oral cosmetic use of a combination of active agents comprising at least taurine, a derivative thereof and/or an acceptable salt thereof, at least vitamin B8 or a derivative thereof and at least a grape extract, for increasing and/or maintaining the thickness of hair fibres and/or limiting the thinning of hair fibres.

Thus, according to a first aspect, the present invention relates to the oral cosmetic use of a combination of active agents comprising at least taurine, a compound chosen from 4-(2-hydroxyethyl)-1-piperazineethanesulfonic acid, N-acyltaurine, acetylhomotaurine, N,N-diacetylsalicyloxyethyltaurine, acylmethyltaurine, homo taurine and hypotaurine and/or an acceptable salt thereof, at least vitamin B8, a stereoisomer or a salt thereof, at least vitamin C in a content of between 2% and 10% by weight, relative to the total weight of the composition, at least a grape extract, and zinc or a salt thereof, for increasing and/or maintaining the thickness of hair fibres and/or limiting the thinning of hair fibres.

Taurine is already described in the prior art, either as a cellular activator for regulating hair cells, proposed for the purposes of hair stimulants for topical application (WO 02/24189), or as an agent that is useful for improving hair density by treatment or prevention of alopecia, via oral administration (WO 2004/000 293).

To the inventors' knowledge, it has hitherto never been proposed or suggested that the oral administration, to an individual in need thereof, of a combination of taurine, a derivative thereof and/or an acceptable salt thereof, vitamin B8 or a derivative thereof and at least one grape extract, as active agents, could prove to be particularly effective for increasing and/or maintaining the thickness of hair fibres, and/or limiting the thinning of hair fibres.

More particularly, the oral administration of a combination of active agents in accordance with the invention can improve the stylability of the head of hair, and/or its hold and manageability and/or improve the volume of the head of hair.

More particularly, the oral administration of a combination of active agents in accordance with the invention can improve the tensile strength of the hair, and/or the styling and/or shaping of the hair and/or prevent and/or combat brittle hair and/or improve the vigour of hair fibres and/or improve the disentangling of hair fibres and/or make the hair less electric and/or improve the hairstyle hold between two shampoo washes.

Furthermore, the oral administration of a combination of active agents in accordance with the invention can limit the visibility of the scalp.

Thus, and as demonstrated by the examples, administration for three months of a combination in accordance with the invention can increase the thickness of the hair, and more particularly can:
- restore volume to the head of hair;
- restore tonicity to the hair;
- reduce the fragility of the hair;
- make the hair stronger, give it greater tensile strength;
- make the hair less brittle;
- improve the stylability of the hair;
- improve the disentangling of the hair;
- make the hair less flyaway, i.e. less electric; and/or
- make the scalp less visible.

According to one embodiment, the grape extract according to the invention is a grape seed extract.

According to the invention, the combination according to the invention also comprises, as additional active agent, zinc or a salt thereof, and preferably zinc gluconate.

The present text also describes, the combination in accordance with the invention also comprises, as additional active agents, vitamins, preferably other than vitamin B8. According to the invention, the vitamin is especially vitamin C.

According to one embodiment, the combination in accordance with the invention may be used in a composition, especially a cosmetic composition, suitable for oral administration.

A cosmetic composition in accordance with the invention offers the same advantages as those afforded by the combination in accordance with the invention, as indicated previously.

According to yet another of the aspects of the present invention, a subject thereof is a cosmetic process for increasing and/or maintaining the thickness of hair fibres, and/or limiting the thinning of hair fibres, comprising at least the oral administration, to an individual, of a combination of active agents or of a composition in accordance with the invention.

A use of a combination of active agents in accordance with the invention is necessarily performed in an effective amount, i.e. an amount that allows the active agents to manifest their properties with regard to the improvement to be afforded to the quality of the head of hair as defined previously.

### Taurine, derivatives thereof and acceptable salts thereof

As active agents that are present in a combination in accordance with the invention, use is made of taurine and/or of a derivative thereof and/or an acceptable salt thereof.

Taurine, or 2-aminoethanesulfonic acid, is an amino acid derivative. It is naturally and ubiquitously present in the human body.

It is involved, for example, in the mechanism of fat digestion since it is present in the structure of bile acids (taurocholic acid and taurochenodeoxycholic acid) of bile salt precursors charged with emulsifier (in the form of micelles) of dietary fats (especially including cholesterol) arriving into the duodenum after a meal.

For the purposes of the present invention, the term "taurine derivatives" means structural analogues of taurine, metabolites thereof or acceptable salts thereof.

Such structural analogues are, for example, 4-(2-hydroxyethyl)-1-piperazineethanesulfonic acid, N-acyltaurine, acetylhomotaurine, N,N-diacetylsalicyloxyethyltaurine, acylmethyltaurine, homo taurine or salts thereof as described in *"*Taurine analogues; a new class of therapeutics: retrospect and prospects", Gupta et al., S. Curr. Med. Chem. 2005; 12(17): 2021-39.

A taurine metabolite is, for example, hypotaurine.

The term "acceptable salts" means salts chosen for their total harmlessness, insofar as the compositions in accordance with the invention are intended to be administered to an individual. Alkali metal or alkaline-earth metal salts, in particular magnesium salts, manganese, iron(II) or zinc salts are most particularly suitable for the invention in this respect.

Taurine derivatives may also be natural extracts rich in taurine (2-aminoethanesulfonic acid) or derivatives thereof.

According to the invention, use will preferably be made of taurine or an acceptable salt thereof.

According to a preferred embodiment, taurine and/or hypotaurine will be used.

According to the invention, taurine, a derivative thereof and/or an acceptable salt thereof are used at daily doses ranging from 1 to 3000 mg per day, advantageously from 5 to 2000 mg per day and preferably from 10 to 300 mg per day.

According to a preferred embodiment, the daily dose is from about 50 to 150 mg per day.

The indicated doses of taurine derivatives or of a salt thereof in the present description are the doses as taurine equivalent.

### Vitamin B8

Vitamin B8, also known as vitamin H, vitamin B7, biotin or coenzyme R, is a molecule having the following structure:

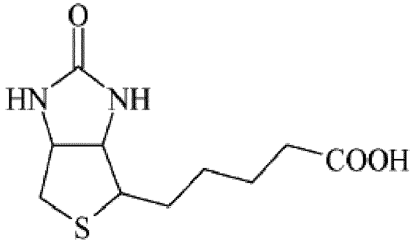

It is water-soluble, heat-stable and UV-sensitive. It is a coenzyme which participates in the metabolism of fatty acids, carbohydrates and amino acids.

For the purposes of the invention, the term "biotin" covers the eight isomers of the preceding formula liable to be used in an isolated form or in the form of mixtures.

As a preferred isomer, mention may be made more particularly of the isomer D-(+)-biotin.

Many biotin derivatives are known to date.

For the purposes of the invention, the biotin derivatives cover both the 20 different stereoisomeric forms of the formula of biotin and also biotin salts.

According to a preferred embodiment, the daily dose is from about 0.005 to 0.06 mg per day.

### Grape extract

A grape extract is naturally rich in polyphenols.

The polyphenols of the grape extracts in accordance with the invention may be in the form of monomers and/or of oligomers. The polyphenols that are more particularly present in grape extracts, especially in grape seed extracts, are polyphenols of the stilbenoid or tannin family, more particularly condensed tannins. They are more particularly oligo-proanthocyanidin (OPC) polyphenols.

A grape extract in accordance with the invention preferably consists of a grape seed extract.

A grape extract in accordance with the invention is used so as to afford daily doses of polyphenols ranging from 0.1 to 2000 mg per day, advantageously from 10 to 1000 mg per day and preferably from 50 to 500 mg per day.

According to a preferred embodiment, the daily dose is from about 50 to 300 mg per day.

Thus, a combination of active agents in accordance with the invention advantageously comprises taurine or hypotaurine, vitamin B8 and a grape seed extract.

In the context of the present invention, the doses of grape extract represent the doses of polyphenols.

### Additional active agent(s)

A combination in accordance with the invention may also comprise one or more additional cosmetic active agent(s).

Advantageously, such an additional cosmetic active agent may be intended to reinforce the desired cosmetic effect as previously described.

As additional active agents that may be used, mention may be made of:
- vitamins, other than vitamin B8, a stereoisomer or a salt thereof, and vitamin C, such as vitamins A, B₅, B₆, or PP (vitamin B₃ or niacin),
- antioxidants, such as curcuminoids; carotenoids, especially a carotenoid chosen from β-carotene, astaxanthin, zeaxanthin and lutein or compounds containing same, such as wolfberry or lactowolfberry; polyphenol compounds, flavonoids such as catechins; proanthocyanidins, anthocyanins, PCOs (procyanidol oligomers); ubiquinones; coffee extracts containing polyphenols and/or diterpenes; chicory extracts; *Ginkgo biloba* extracts; grape extracts rich in proanthocyanidins; pimento extracts; soybean extracts; cocoa or coconut milk; pomegranate; Emblica,
- minerals other than zinc, such as calcium, magnesium, copper, iron, iodine, manganese, selenium and chromium (III),
- sugars,
- amino acids, especially sulfur amino acids such as glutathione precursors, selenium amino acids, citrulline,
- prebiotics, chosen especially from oligosaccharides, produced from glucose, galactose, xylose, maltose, sucrose, lactose, starch, xylan, hemicellulose, inulin, gums of acacia type, for example, or a mixture thereof. More particularly, the oligosaccharide comprises at least one fructo-oligosaccharide. More particularly, this prebiotic may comprise a mixture of fructo-oligosaccharide and of inulin.
- phytosterols, such as resveratrol,
- hesperidin and neohesperidin,
- orthosilicic acid, monomethylsilanetriol, and
- mixtures thereof.

A combination in accordance with the invention comprises zinc or a salt thereof as active agent, preferably zinc gluconate.

A combination in accordance with the invention comprises vitamin C.

The present text describes a combination that comprises, besides taurine, a derivative thereof and/or an acceptable salt thereof, vitamin B8 or a derivative thereof, and a grape extract as defined previously, zinc or a salt thereof, and vitamins other than vitamin B8 or a derivative thereof, preferably vitamin C.

A combination in accordance with the invention comprises, besides taurine, a compound chosen from 4-(2-hydroxyethyl)-1-piperazineethanesulfonic acid, N-acyltaurine, acetylhomotaurine, N,N-diacetylsalicyloxyethyltaurine, acylmethyltaurine, homotaurine and hypotaurine and/or an acceptable salt thereof, vitamin B8, a stereoisomer or a salt thereof, and a grape extract as defined previously, zinc or a salt thereof, and vitamin C in a content of between 2% and 10% by weight, relative to the total weight of the composition.

According to one embodiment, a combination of the invention may comprise additional hydrophilic active agents. Hydrophilic active agents that may be used include proteins or protein hydrolysates, amino acids, polyols, especially of C2 to C10, for instance glycerol, sorbitol, butylene glycol or polyethylene glycol, urea, allantoin, sugars and sugar derivatives, water-soluble vitamins, starch, and bacterial or plant extracts, for instance those from *Aloe vera.*

According to another embodiment, a combination of the invention may also comprise a lipophilic active agent. Lipophilic active agents that may be used include retinol (vitamin A) and derivatives thereof, ceramides and essential oils.

The present text also describes a combination that comprises, besides taurine or a derivative thereof, vitamin B8 or a derivative thereof and a grape seed extract as defined previously, zinc gluconate and optionally vitamin C.

According to a preferred embodiment, a combination in accordance with the invention comprises, besides taurine, a compound chosen from 4-(2-hydroxyethyl)-1-piperazineethanesulfonic acid, N-acyltaurine, acetylhomotaurine, N,N-diacetylsalicyloxyethyltaurine, acylmethyltaurine, homo taurine and hypotaurine and/or an acceptable salt thereof, vitamin B8, a stereoisomer or a salt thereof, and a grape extract as defined previously, zinc gluconate and vitamin C in a content of between 2% and 10% by weight, relative to the total weight of the composition.

### Composition

According to one aspect of the invention, a combination of active agents in accordance with the invention may be used in a cosmetic composition suitable for oral administration.

A composition in accordance with the invention comprises a physiologically or pharmaceutically acceptable vehicle.

Needless to say, a person skilled in the art will take care to select the additional active agents and the amount thereof such that the advantageous properties of the composition in accordance with the invention are not, or are not substantially, adversely affected by the envisaged addition.

A cosmetic composition in accordance with the invention may comprise taurine, a derivative thereof and/or an acceptable salt thereof, in a content of between 1% and 30% by weight relative to the total weight of the composition, expressed as taurine equivalent.

Preferably, taurine, a derivative thereof and/or an acceptable salt thereof is present in a content of between 5% and 20% by weight and preferentially between 10% and 15% by weight relative to the total weight of the composition, expressed as taurine equivalent.

Vitamin B8 or a derivative thereof in accordance with the invention may be used in a composition in accordance with the invention in a content of between 0.0001% and 1% by weight relative to the total weight of the composition.

Preferably, vitamin B8 may be used in a content of between 0.0005% and 0.5% by weight and preferentially between 0.001% and 0.1% by weight relative to the total weight of the composition.

In addition, a cosmetic composition in accordance with the invention comprises a grape extract, in particular a grape seed extract, as defined previously, in a content of between 1% and 80% by weight relative to the total weight of the composition, expressed as dose of polyphenols.

Preferably, the grape extract is present in a content of between 5% and 70% by weight, preferentially between 10% and 60% by weight and preferably between 10% and 30% by weight, relative to the total weight of the composition, expressed as dose of polyphenols.

A composition in accordance with the invention may also comprise the additional cosmetic active agents indicated previously. They may be present in a composition in accordance with the invention in a content of between 0.0001% and 20% by weight relative to the total weight of the composition.

More particularly, zinc or a salt thereof may be used in a composition in accordance with the invention in a content of between 0.1% and 30% by weight relative to the total weight of the composition.

Preferably, zinc or a salt thereof may be used in a content of between 0.5% and 20% by weight and preferentially between 1% and 10% by weight relative to the total weight of the composition.

Furthermore, the vitamin C may be used in a composition in a content of between 0.5% and 25% by weight relative to the total weight of the composition. The vitamin C may be used in a content of between 1% and 20% by weight and even between 2% and 10% by weight relative to the total weight of the composition.

According to the invention, the vitamin C is used in a composition in accordance with the invention in a content of between 2% and 10% by weight relative to the total weight of the composition.

A combination of active agents and a composition in accordance with the invention make it possible, via their oral administration, to increase and/or maintain the thickness of hair fibres, and/or to limit the thinning of hair fibres, and thus to afford all of the advantages mentioned previously.

According to one embodiment, a combination of active agents and a composition in accordance with the invention make it possible to improve the stylability of the head of hair, and/or its hold and manageability and/or to improve the volume of the head of hair.

According to one embodiment, a combination of active agents and a composition in accordance with the invention can improve the tensile strength of the hair, and/or the styling and/or shaping of the hair and/or prevent and/or combat brittle hair and/or improve the vigour of hair fibres and/or improve the disentangling of hair fibres and/or make the hair less electric and/or improve the hairstyle hold between two shampoo washes.

According to one embodiment, a combination of active agents and a composition in accordance with the invention can limit the visibility of the scalp.

The combination of active agents in accordance with the invention or the composition in accordance with the invention is administered orally.

The combinations and compositions in accordance with the invention, intended for oral administration, may especially comprise all or only a part of the daily dose.

The required daily dose may thus be split so as to be taken, for example, 1 to 3 times in the day.

Typically, the duration of this cosmetic treatment may be greater than 4 weeks, especially from 4 to 15 weeks, with, where appropriate, one or more periods of stoppage.

The oral route has the advantage of acting more globally on the entire structure of the hair.

The term "oral-route cosmetic composition" means, for example, nutritional, nutraceutical or cosmeceutical compositions, comprising at least one combination according to the invention.

In the case of compositions suitable for oral administration, the use of an ingestible support is preferred. The ingestible support may be of diverse nature according to the type of composition under consideration.

For ingestion, numerous embodiments of oral compositions and in particular of food supplements are possible.

Such compositions may be formulated via any usual process known to those skilled in the art.

Thus, a composition in accordance with the invention may preferably take the form of a sugar-coated tablet, a gel capsule, a suspension, a gel, an emulsion, an oral solution, a tablet to be swallowed or chewed, a soft capsule, a granule to be dissolved, a syrup, a lozenge or a food supplement.

In particular, a combination of active agents in accordance with the invention may be incorporated into any form of food supplement or enriched food, for example food bars or compact or loose powders. The powders may be diluted with water, in soda, dairy products or soybean derivatives, or may be incorporated into food bars.

According to a preferred embodiment, a composition in accordance with the invention administered orally may be formulated in the form of a sugar-coated tablet, a gel capsule, a gel, an emulsion, a tablet, a capsule, a hydrogel, a food bar, a compact or loose powder, a liquid suspension or solution, a confectionery product, a fermented milk, a fermented cheese, a chewing gum, a toothpaste or a spray solution.

Milk, yoghurt, cheese, fermented milks, milk-based fermented products, ice creams, fermented or unfermented cereal-based products, milk-based powders, infant and baby formulae, animal feed in particular for pets, tablets or lozenges, liquid bacterial suspensions, oral supplements in dry form and oral supplements in liquid form are suitable, for example, as food supports.

The oral compositions may be either in anhydrous form or in aqueous form.

A combination of active agents in accordance with the invention may be formulated with the usual excipients and components for such oral compositions or food supplements, namely, in particular, fatty and/or aqueous components, humectants, thickeners, preserving agents, texturing agents, taste agents and/or coating agents, antioxidants, fillers, anticaking agents, disintegrants, lubricants, preserving agents and dyes that are common in the food sector.

The formulating agents and excipients for an oral composition, and in particular for food supplements, are known in this field and are not the subject of a detailed description herein.

In particular, a composition in accordance with the invention may be a food composition for human consumption. Such compositions may be, in particular, nutritional whole foods, drinks, mineral waters, soups, dietary supplements and food replacement supplements, nutritional bars, confectionery, milk-based products or fermented milk-based products, yoghurts, milk-based powders, enteral nutritional products, infant and/or baby compositions, fermented or unfermented cereal-based products, ice creams, chocolate, coffee, "culinary" products such as mayonnaise, tomato puree or salad dressings.

### Process

According to another of its aspects, the present invention relates to a cosmetic process for improving the quality of the head of hair, especially for increasing and/or maintaining the thickness of hair fibres, and/or limiting the thinning of hair fibres, characterized in that it comprises at least the oral administration, to said individual, of a combination or of a composition in accordance with the invention.

Advantageously, the application of a process of the invention offers the advantages indicated previously as being associated with the use of a combination or of a composition in accordance with the invention.

A cosmetic process according to the invention may especially be performed by administration of a food composition as defined above.

A process of the invention may be performed on a daily basis for example, at a rate of, for example, a single administration per day or an administration twice a day, for example once in the morning and once in the evening, or three times a day, in particular at each meal.

A cosmetic process according to the invention may be performed, for example, by daily administration of a composition formulated, for example, in the form of gel capsules, sugar-coated tablets, emulsions, tablets, capsules or oral vials, in appropriate amount and number, depending on their form.

An effective amount of a combination in accordance with the invention may be administered in a single dose per day or in fractional doses over the course of the day, for example two or three times per day.

A process according to the invention may advantageously comprise a single administration.

A cosmetic process may be performed over a time period ranging from one week to several weeks, or even several months, this period moreover possibly being repeated after periods without treatment, for several months or even several years.

By way of example, the administration of a combination of active agents in accordance with the invention may be performed, for example, at a rate of three times a day, generally over a prolonged period of at least four weeks, or even four to fifteen weeks, optionally comprising one or more periods of stoppage, or being repeated after a period of stoppage.

Needless to say, the present invention may benefit both men and women.

In the description and in the examples that follow, unless otherwise mentioned, the percentages are weight percentages and the ranges of values written in the form "between ... and ..." include the stated lower and upper limits. The ingredients are mixed, before being formed, in the order and under conditions that may be readily determined by those skilled in the art.

The examples below are presented as non-limiting illustrations of the field of the invention.

### EXAMPLES

### Example 1

### Oral composition in tablet form

| **Ingredients** | (mg/tablet) | (weight percentage relative to the total weight of the composition) |
|---|---|---|
| Taurine⁽¹⁾ | 75 | 10.641 |
| Zinc gluconate⁽²⁾ | 25.74 | 3.652 |
| Grape seed extract⁽³⁾ | 93.5 | 13.266 |
| Vitamin B8⁽⁴⁾ | 0.016 | 0.002 |
| Vitamin C⁽⁵⁾ | 18.34 | 2.602 |
| | | |

| **Core excipients** | | |
|---|---|---|
| Dibasic calcium phosphate dihydrate | 218 | 30.931 |
| Microcrystalline cellulose | 214 | 30.363 |
| Croscarmellose sodium | 20 | 2.838 |
| Silicon dioxide | 7 | 0.993 |
| Magnesium stearate | 6 | 0.851 |
| | | |

| **Film-coating agents** | | |
|---|---|---|
| Hydroxypropylmethylcellulose | 9.18 | 1.303 |
| Hydroxypropylcellulose | 9.18 | 1.303 |
| Talc | 5.44 | 0.772 |
| Titanium dioxide | 3.21 | 0.455 |
| Yellow iron oxide | 0.12 | 0.017 |
| Red iron oxide | 0.07 | 0.010 |

| | | |
|---|---|---|
| (1) Sold under the name Taurine by the company Quimdis. (2) Sold under the name Gluconal Zn-P by the company Arnaud. (3) Sold under the name Vitaflavan 50 by the company DRT. (4) Sold under the name D-Biotin by the company DSM. (5) Sold under the name Ascorbic Acid 90% Granulation by the company DSM. | | |

One to three of these capsules may be taken per day.

This composition may be prepared according to the process described below:
D-biotin and the excipients are first mixed together. Taurine, zinc gluconate, the grape seed extract and vitamin C are then added to this mixture.

The mixture thus obtained is subsequently lubricant heated and then compressed, before being coated using film-forming agents and purified water.

### Example 2

An observational study without a control group was performed, on the basis of the composition of Example 1 under dermatological control, by 16 dermatologists, on 113 women in good health, from 18 to 60 years old, complaining of limp, fine hair lacking tonicity and volume.

These women were supplemented for 3 months with the composition of Example 1 at a rate of 2 tablets per day.

The efficacy of the supplementation was then validated by the dermatologists:
- the day of inclusion (V1D0 - for visit 1, day 0);
- then 30 days, 60 days and 90 days later.

The evaluations are performed on a 10-point grading scale, namely:

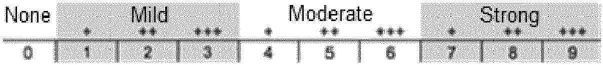

Two observations were also made by professional hairstylists:
- in the 5 days following the first visit of the dermatologists;
- in the 10 days following the visit of the dermatologists at 90 days.

The evaluations by these hairstylists are also performed on a 10-point grading scale equivalent to that used by the dermatologists.

The evaluations were thus performed after 30, 60 and 90 days of supplementation. The individuals had to fill in a questionnaire on the following 5-point grading scale: Entirely agree, Agree, Do not agree, Do not at all agree, No opinion.

Table I below summarizes the changes in the parameters evaluated by the 16 dermatologists.

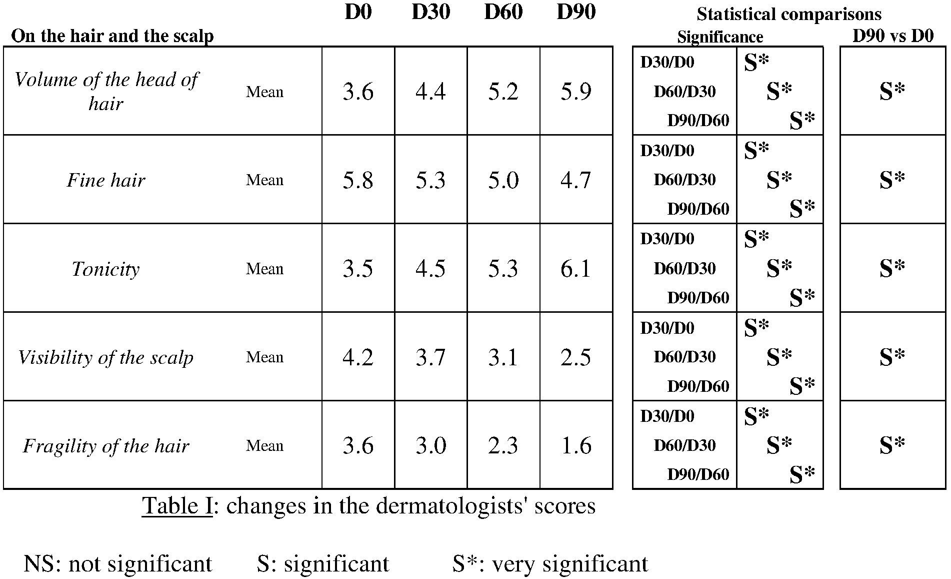

From D30, the dermatologists' scores show a statistically significant improvement in the fine nature of the hair, the fragility of the hair and the volume and tonicity of the head of hair. A decrease in the visibility of the scalp may also be noted. This improvement is gradual throughout the taking of the test product.

Table II below summarizes the changes in the evaluations by the individuals.

The percentage of individuals with a favourable opinion increases over time, reflecting a real physiological effect of the test product. By way of example, after 3 months of supplementation, 68% of the individuals find that their hair is thicker, and 81% find that their hair is easier to style.

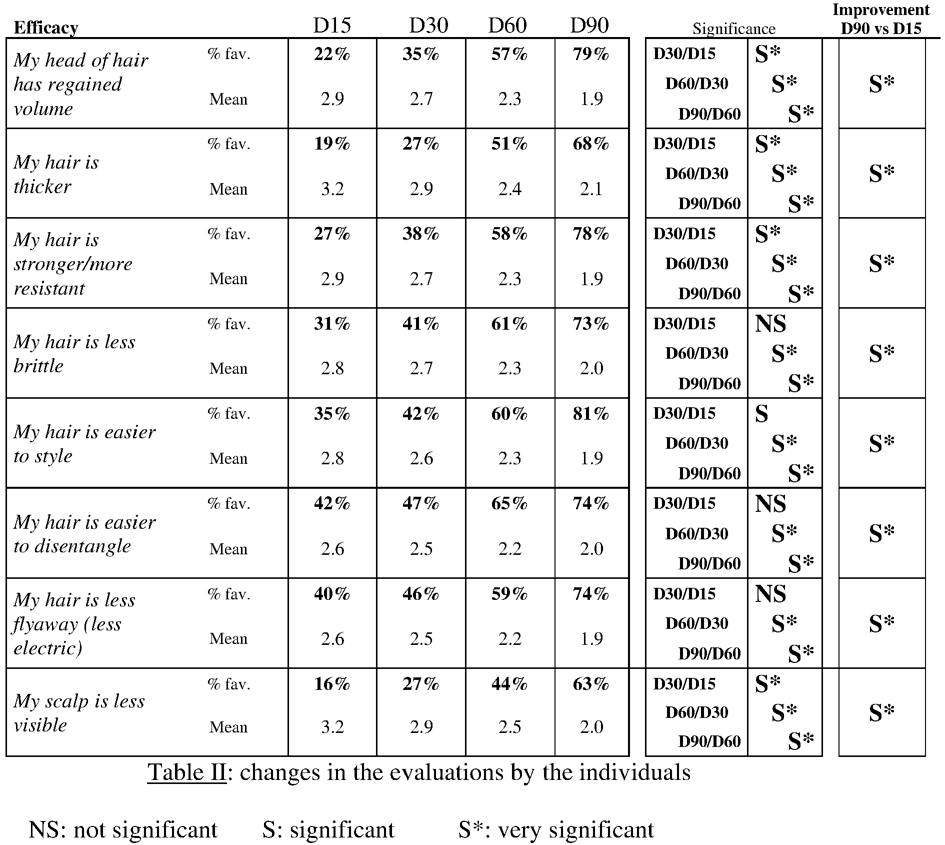

Table III below summarizes the evaluations made by the professional experts (hairstylists).

After 3 months of supplementation, the hairstylists note a statistically significant improvement in the volume of the head of hair, the thickness of the hair and the fragility of the hair, and also improvement in the stylability.

**Table III: professional evaluations (hairstylists)**

| | | | | |
|---|---|---|---|---|
| *Volume of the head of hair* | Mean | **4** | **5.7** | **S^{∗}** |
| *Thickness of the hair* | Mean | **3.5** | **5** | **S^{∗}** |
| *Stylability* | Mean | **4.1** | **5.9** | **S^{∗}** |
| *Brittle hair*^{∗} | Mean | **3.9** | **2.3** | **S^{∗}** |
| *Fragile hair* (*fine and fragile*) | Mean | **4.9** | **3.3** | **S^{∗}** |
| *Anaemic hair* (*fragile, brittle hair lacking keratin*) | Mean | **4.4** | **2.5** | **S^{∗}** |
| *Visibility of the scalp* | Mean | **3.6** | **2.4** | **S^{∗}** |

| | | | | |
|---|---|---|---|---|
| NS: not significant S: significant S*: very significant | | | | |

In agreement with the dermatologists and the women participating in the study, the hairstylists evaluated that the test product especially improved the volume of the head of hair, the thickness of the hair and the stylability, and made it possible to limit the fragility of the hair.

## Claims

1. Oral cosmetic use of a composition comprising a combination of active agents comprising:
at least taurine, a compound chosen from 4-(2-hydroxyethyl)-1-piperazineethanesulfonic acid, N-acyltaurine, acetylhomotaurine, N,N-diacetylsalicyloxyethyltaurine, acylmethyltaurine, homo taurine and hypotaurine and/or an acceptable salt thereof,
at least vitamin B8, a stereoisomer or a salt thereof
at least vitamin C in a content of between 2% and 10% by weight, relative to the total weight of the composition,
at least a grape extract, and
zinc or a salt thereof,
for increasing and/or maintaining the thickness of hair fibres and/or limiting the thinning of hair fibres.

2. Use according to Claim 1, for improving the stylability of the head of hair, and/or its hold and manageability and/or improving the volume of the head of hair.

3. Use according to Claim 1, for improving the tensile strength of the hair, and/or the styling and/or shaping of the hair and/or preventing and/or combating brittle hair and/or improving the vigour of hair fibres and/or improving the disentangling of hair fibres and/or making the hair less electric and/or improving the hairstyle hold between two shampoo washes.

4. Use according to Claim 1, for limiting the visibility of the scalp.

5. Use according to any one of the preceding claims, in which the grape extract is a grape seed extract.

6. Use according to any one of the preceding claims, **characterized in that** the combination comprises zinc gluconate.

7. Use according to any one of the preceding claims, in which composition is a cosmetic composition preferably being able to take the form of a sugar-coated tablet, a gel capsule, a suspension, a gel, an emulsion, an oral solution, a tablet to be swallowed or chewed, a soft capsule, a granule to be dissolved, a syrup, a lozenge or a food supplement.

8. Use according to Claim 7, in which taurine, a compound chosen from 4-(2-hydroxyethyl)-1-piperazineethanesulfonic acid, N-acyltaurine, acetylhomotaurine, N,N-diacetylsalicyloxyethyltaurine, acylmethyltaurine, homotaurine and hypotaurine and/or an acceptable salt thereof is used in a content of between 1% and 30% by weight, preferably between 5% and 20% by weight and preferentially between 10% and 15% by weight relative to the total weight of the composition, expressed as taurine equivalent.

9. Use according to Claim 7 or 8, in which vitamin B8, a stereoisomer or a salt thereof is used in a content of between 0.0001% and 1% by weight, preferably between 0.0005% and 0.5% by weight and preferentially between 0.001% and 0.1% by weight relative to the total weight of the composition.

10. Use according to any one of Claims 7 to 9, in which the grape extract is used in a content of between 1% and 80% by weight, preferably between 5% and 70% by weight, preferentially between 10% and 60% by weight and preferably between 10% and 30% by weight, relative to the total weight of the composition, expressed as dose of polyphenols.

11. Use according to any one of Claims 6 to 10, in which the composition comprises zinc or a salt thereof, preferably zinc gluconate, used in a content of between 0.1% and 30% by weight, preferably between 0.5% and 20% by weight and preferentially between 1% and 10% by weight relative to the total weight of the composition.

12. Cosmetic process for increasing and/or maintaining the thickness of hair fibres, and/or limiting the thinning of hair fibres, comprising at least the oral administration, to an individual, of a combination of active agents or of a composition as defined in any one of Claims 1 to 11.

## Patentansprüche

1. Orale kosmetische Verwendung einer Zusammensetzung, die eine Kombination von Wirkstoffen enthält, umfassend:
mindestens Taurin, eine Verbindung, ausgewählt aus 4-(2-Hydroxyethyl)-1-piperazinethansulfonsäure, N-Acyltaurin, Acetylhomotaurin, N,N-Diacetylsalicyloxyethyltaurin, Acylmethyltaurin, Homotaurin und Hypotaurin und/oder ein akzeptables Salz davon,
mindestens Vitamin B8, ein Stereoisomer oder ein Salz davon,
mindestens Vitamin C in einem Gehalt zwischen 2 und 10 Gew.-%, bezogen auf das Gesamtgewicht der Zusammensetzung,
mindestens einen Traubenextrakt, und
Zink oder ein Salz davon,
um die Dicke von Haarfasern zu erhöhen und/oder zu erhalten und/oder die Ausdünnung von Haarfasern zu begrenzen.

2. Verwendung nach Anspruch 1 zur Verbesserung der Frisierbarkeit des Kopfhaares und/oder des Haltes und der Handhabbarkei und/oder zur Verbesserung des Volumens des Kopfhaares.

3. Verwendung nach Anspruch 1 zur Verbesserung der Reißfestigkeit des Haares und/oder des Stylings und/oder der Formung des Haares und/oder zur Vorbeugung und/oder Bekämpfung von brüchigem Haar und/oder zur Verbesserung der Spannkraft von Haarfasern und/oder zur Verbesserung der Entwirrung von Haarfasern und/oder um das Haar weniger elektrisch zu machen und/oder zur Verbesserung des Haltes der Frisur zwischen zwei Haarwäschen.

4. Verwendung nach Anspruch 1, um die Sichtbarkeit der Kopfhaut zu begrenzen.

5. Verwendung nach einem der vorhergehenden Ansprüche, bei der der Traubenextrakt ein Traubenkernextrakt ist.

6. Verwendung nach einem der vorhergehenden Ansprüche, **dadurch gekennzeichnet, dass** die Kombination Zinkgluconat enthält.

7. Verwendung nach einem der vorhergehenden Ansprüche, bei der die Zusammensetzung eine kosmetische Zusammensetzung ist, die vorzugsweise die Form einer mit Zucker beschichteten Tablette, einer Gelkapsel, einer Suspension, eines Gels, einer Emulsion, einer oralen Lösung, einer Schluck- oder Kautablette, einer Weichkapsel, eines Granulats zum Auflösen, eines Sirups, einer Pastille oder eines Nahrungsergänzungsmittels annehmen kann.

8. Verwendung nach Anspruch 7, bei der Taurin, eine Verbindung, ausgewählt aus 4-(2-Hydroxyethyl)-1-Piperazinethansulfonsäure, N-Acyltaurin, Acetylhomotaurin, N,N-Diacetylsalicyloxyethyltaurin, Acylmethyltaurin, Homotaurin und Hypotaurin und/oder ein akzeptables Salz davon, in einem Gehalt zwischen 1 und 30 Gew.-%, vorzugsweise zwischen 5 und 20 Gew.-% und bevorzugt zwischen 10 und 15 Gew.-%, bezogen auf das Gesamtgewicht der Zusammensetzung, ausgedrückt als Taurinäquivalent, verwendet wird.

9. Verwendung nach Anspruch 7 oder 8, bei der Vitamin B8, ein Stereoisomer oder ein Salz davon, in einem Gehalt zwischen 0,0001 und 1 Gew.-%, vorzugsweise zwischen 0,0005 und 0,5 Gew.-% und bevorzugt zwischen 0,001 und 0,1 Gew.-%, bezogen auf das Gesamtgewicht der Zusammensetzung, verwendet wird.

10. Verwendung nach einem der Ansprüche 7 bis 9, bei der Traubenextrakt in einem Gehalt zwischen 1 und 80 Gew.-%, vorzugsweise zwischen 5 und 70 Gew.-%, bevorzugt zwischen 10 und 60 Gew.-% und vorzugsweise zwischen 10 und 30 Gew.-%, bezogen auf das Gesamtgewicht der Zusammensetzung, ausgedrückt als Dosis von Polyphenolen, verwendet wird.

11. Verwendung nach einem der Ansprüche 6 bis 10, bei der die Zusammensetzung Zink oder ein Salz davon, vorzugsweise Zinkgluconat, enthält, welches in einem Gehalt zwischen 0,1 und 30 Gew.-%, vorzugsweise zwischen 0,5 und 20 Gew.-% und bevorzugt zwischen 1 und 10 Gew.-%, bezogen auf das Gesamtgewicht der Zusammensetzung, verwendet wird.

12. Kosmetisches Verfahren zur Erhöhung und/oder Erhaltung der Dicke von Haarfasern und/oder zur Begrenzung der Ausdünnung von Haarfasern, umfassend mindestens die orale Verabreichung einer Wirkstoffkombination oder einer Zusammensetzung nach einem der Ansprüche 1 bis 11 an ein Individuum.

## Revendications

1. Utilisation cosmétique par voie orale d'une composition comprenant une association de principes actifs comprenant :
au moins de la taurine, un composé choisi parmi l'acide 4-(2-hydroxyéthyl)-1-pipérazineéthanesulfonique, la N-acyltaurine, l'acétylhomotaurine, la N,N-diacétylsalicyloxyéthyltaurine, l'acylméthyltaurine, l'homotaurine et l'hypotaurine et/ou l'un de leurs sels acceptables,
au moins de la vitamine B8, l'un de ses stéréoisomères ou l'un de ses sels
au moins de la vitamine C en une teneur comprise entre 2 % et 10 %en poids, par rapport au poids total de la composition,
au moins un extrait de raisins, et
du zinc ou l'un de ses sel,
pour augmenter et/ou maintenir l'épaisseur des fibres capillaires et/ou limiter l'affinement des fibres capillaires.

2. Utilisation selon la revendication 1, pour améliorer la coiffabilité de la chevelure, et/ou sa tenue et sa gestion et/ou pour améliorer le volume de la chevelure.

3. Utilisation selon la revendication 1, pour améliorer la résistance à la traction des cheveux, et/ou le coiffage et/ou la mise en forme des cheveux et/ou prévenir et/ou lutter contre les cheveux cassants et/ou améliorer la vigueur des fibres capillaires et/ou améliorer le démêlage des fibres capillaires et/ou rendre les cheveux moins électriques et/ou améliorer la tenue de la coiffure entre deux shampooings.

4. Utilisation selon la revendication 1, pour limiter la visibilité du cuir chevelu.

5. Utilisation selon l'une quelconque des revendications précédentes, dans laquelle l'extrait de raisins est un extrait de pépins de raisins.

6. Utilisation selon l'une quelconque des revendications précédentes, **caractérisée en ce que** l'association comprend du gluconate de zinc.

7. Utilisation selon l'une quelconque des revendications précédentes, dans laquelle la composition est une composition cométique pouvant de préférence prendre la forme d'une dragée, d'une gélule, d'une suspension, d'un gel, d'une émulsion, d'une solution buvable, d'un comprimé à avaler ou à croquer, d'une capsule molle, d'un granulé à dissoudre, d'un sirop, d'une pastille ou d'un complément alimentaire.

8. Utilisation selon la revendication 7, dans laquelle la taurine, un composé choisi parmi l'acide 4-(2-hydroxyéthyl)-1-pipérazineéthanesulfonique, la N-acyltaurine, l'acétylhomotaurine, la N,N-diacétylsalicyloxyéthyltaurine, l'acylméthyltaurine, l'homotaurine et l'hypotaurine et/ou l'un de leurs sels acceptables, est utilisée en une teneur comprise entre 1 % et 30 % en poids, de préférence entre 5 % et 20 % en poids et préférentiellement entre 10 % et 15 % en poids, par rapport au poids total de la composition, exprimée en équivalent taurine.

9. Utilisation selon la revendication 7 ou 8, dans laquelle la vitamine B8, l'un de ses stéréoisomère ou l'un de ses sels, est utilisée en une teneur comprise entre 0,0001 % et 1 % en poids, de préférence entre 0,0005 % et 0,5 % en poids et préférentiellement entre 0,001 % et 0,1 % en poids, par rapport au poids total de la composition.

10. Utilisation selon l'une quelconque des revendications 7 à 9, dans laquelle l'extrait de raisins est utilisé en une teneur comprise entre 1 % et 80 % en poids, de préférence entre 5 % et 70 % en poids, préférentiellement entre 10 % et 60 % en poids et de préférence entre 10 % et 30 % en poids, par rapport au poids total de la composition, exprimé en dose de polyphénols.

11. Utilisation selon l'une quelconque des revendications 6 à 10, dans laquelle la composition comprend du zinc ou l'un de ses sels, de préférence du gluconate de zinc, utilisé en une teneur comprise entre 0,1 % et 30 % en poids, de préférence entre 0,5 % et 20 % en poids et préférentiellement entre 1 % et 10 % en poids, par rapport au poids total de la composition.

12. Procédé cosmétique pour augmenter et/ou maintenir l'épaisseur des fibres capillaires, et/ou limiter l'affinement des fibres capillaires, comprenant au moins l'administration par voie orale, à un individu, d'une association de principes actifs ou d'une composition telle que définies dans l'une quelconque des revendications 1 à 11.
